# EUROPEAN PATENT APPLICATION

(11) **EP 3 266 909 A1**
(43) Date of publication of application: **10.01.2018**
(21) Application number: 17177192.6
(22) Date of filing: 11.06.2009
(51) Int. Cl.: D01F 6/62, D01F 1/10, D06M 15/277, D06M 15/576

(54) **MELT BLOWN FINE FIBERS AND METHODS OF MANUFACTURE**

(30) Priority: 12.06.2008 US 61091 P
(62) Divisional of application: 09763637.7
(71) Applicant: 3M Innovative Properties Co., St. Paul, MN 55133-3427 (US)
(72) Inventor: Moore, Eric, M., Saint Paul, MN 55133-3427 (US); Scholz, Matthew, T., Saint Paul, MN 55133-3427 (US); Karls, Korey, W., Saint Paul, MN 55104 (US); Probeni, Francis, E., Saint Paul, MN 55133-3427 (US); Landgrebe, Kevin, D., Saint Paul, MN 55133-3427 (US); Jennen, Jay, M., Saint Paul, MN 55133-3427 (US)
(74) Representative: Vossius & Partner Patentanwälte Rechtsanwälte mbB

(57) **Abstract**

The present invention relates to a single or multi-layer aqueous repellent article formed at least in part of a web of fine fibers, wherein the fine fibers comprise:
one or more thermoplastic aliphatic polyesters; and
a viscosity modifier selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof; and wherein the fine fibers have an average fiber diameter of less than 20 microns; and wherein a repellent additive is incorporated into the melt as the web is made, a repellent additive is coated onto the fibers prior to web formation, or a repellent additive is coated onto the formed or semi-formed web.

## Description

### Cross Reference to Related Applications

This application claims the benefit of U.S. Provisional Patent Application No. 61/061,091, filed June 12, 2008, which is incorporated herein by reference.

### Background

Melt-blowing (or MB) is the process of forming fibers by extruding molten polymer through small orifices surrounded by high speed heated gas jets. This process is also referred to as blown micro fiber (or BMF). The most common thermoplastic material used for the BMF process is polypropylene, which produces a very fine fiber with good thermal stability.

There is a growing interest in replacing petroleum based polymers, such as polypropylene, with resource renewable polymers, i.e. polymers derived from plant based materials. Ideal resource renewable polymers are "carbon dioxide neutral" meaning that as much carbon dioxide is consumed in growing the plants base material as is given off when the product is made and disposed of. Biodegradable materials have adequate properties to permit them to break down when exposed to conditions which lead to composting. Examples of materials thought to be biodegradable include aliphatic polyesters such as poly(lactic acid), poly(glycolic acid), poly(caprolactone), copolymers of lactide and glycolide, poly(ethylene succinate), and combinations thereof.

Difficulty is encountered in the use of aliphatic polyesters such as poly(lactic acid) for BMF due to aliphatic polyester thermoplastics having relatively high melt viscosities which yield nonwoven webs that generally cannot be made at the same fiber diameters that polypropylene can. The coarser fiber diameters of polyester webs can limit their application as many final product properties are controlled by fiber diameter. For example, course fibers lead to a noticeably stiffer and less appealing feel for skin contact applications. Furthermore, course fibers produce webs with larger porosity that can lead to webs that have less of a barrier property, e.g. less repellency to aqueous fluids.

The processing of aliphatic polyesters as microfibers has been described in U.S. Patent No. 6,645,618. U.S. Patent No. 6,111,160 (Gruber et.al.) discloses the use of melt stable polylactides to form nonwoven articles via melt blown and spunbound processes.

U.S. Patent Nos. 5,585,056 and 6,005,019 describe a surgical article comprising absorbable polymer fibers and a plasticizer containing stearic acid and its salts.

### Brief Description of the Drawings

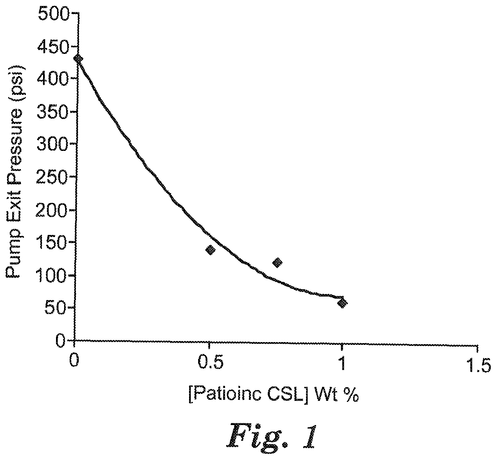

FIGURE 1 is a graph showing the drop in pump exit back pressure with the addition of an exemplary viscosity modifier.

### Disclosure of Invention

The present disclosure is directed to fine fibers of aliphatic polyesters, articles made with the fine fibers, and a method for making the aliphatic polyester fine fibers by a blown microfiber (BMF) process. The fibers may be melt-processable and have utility in a variety of food safety, medical, personal hygiene, disposable and reusable garments, and water purification applications.

A melt blown web of the fine fibers is formed by use of a viscosity modifier to reduce the viscosity of the aliphatic polyesters, such as PLA. In certain preferred embodiments, the viscosity modifier is selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof. By reducing the viscosity of the aliphatic polyester during the BMF process, the average diameter of the fibers is reduced, resulting in fine fibers, typically less than 20 microns, in the melt blown web.

Exemplary aliphatic polyesters are poly(lactic acid), poly(glycolic acid), poly(lactic-co-glycolic acid), polybutylene succinate, polyhydroxybutyrate, polyhydroxyvalerate, blends, and copolymers thereof.

Articles made with the fine fibers comprise molded polymeric articles, polymeric sheets, polymeric fibers, woven webs, nonwoven webs, porous membranes, polymeric foams, layered fine fibers, composite webs such as SMS (Spunbond, Meltblown, Spunbond), SMMS, and combinations thereof made of the fine fibers described herein including thermal or adhesive laminates. Examples of useful articles of this disclosure are wound contact materials made of a film, foam and/or woven or nonwoven comprising the fine fibers and sterilization wraps, surgical drapes or surgical gowns made at least in part of the fine fibers.

Products such as medical gowns, medical drapes, sterilization wraps, wipes, absorbents, insulation, and filters can be made from melt-blown fine fibers of aliphatic polyesters, such as PLA. Films, membranes, nonwovens, scrims and the like can be extrusion bonded or thermally laminated directly to the webs. Due to the diameter of the fine fiber, the webs have a soft feel similar to polyolefin webs but in many cases superior tensile strength due to the higher modulus of the aliphatic polyester used.

The method of the present disclosure comprises providing the aliphatic polyesters and the viscosity modifiers as described herein, and melt blowing these materials sufficiently to yield a web of fine fibers.

In one aspect, the polymer is melt processable, such that the polymer composition is capable of being extruded.

While not intending to be bound by theory, the viscosity modifier likely does not plasticize the melt processable fine fibers of aliphatic polyesters. The compositions are preferably non-irritating and non-sensitizing to mammalian skin and biodegradable. The aliphatic polyester generally has a lower melt processing temperature and can yield a more flexible output material.

For the following defined terms, these definitions shall be applied, unless a different definition is given in the claims or elsewhere in the specification.

The term "biodegradable" means degradable by the action of naturally occurring microorganisms such as bacteria, fungi and algae and/or natural environmental factors such as hydrolysis, transesterification, exposure to ultraviolet or visible light (photodegradable) and enzymatic mechanisms or combinations thereof.

The term "biocompatible" means biologically compatible by not producing toxic, injurious or immunological response in living tissue. Biocompatible materials may also be broken down by biochemical and/or hydrolytic processes and absorbed by living tissue. Test methods used include ASTM F719 for applications where the fine fibers contact tissue such as skin, wounds, mucosal tissue including in an orifice such as the esophagus or urethra, and ASTM F763 for applications where the fine fibers are implanted in tissue.

The term "fine fiber" generally refers to fibers having an average diameter of less than 20 microns, preferably less than 15 microns, more preferably less than 10 microns, and most preferably less than 5 microns.

The recitation of numerical ranges by endpoints includes all numbers subsumed within that range (e.g. 1 to 5 includes 1,1.5, 2, 2.75, 3, 3.8, 4, and 5).

As used in this specification and the appended claims, the singular forms "a", "an", and "the" include plural referents unless the content clearly dictates otherwise. Thus, for example, reference to fine fibers containing "a compound" includes a mixture of two or more compounds. As used in this specification and the appended claims, the term "or" is generally employed in its sense including "and/or" unless the content clearly dictates otherwise.

Unless otherwise indicated, all numbers expressing quantities or ingredients, measurement of properties and so forth used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings of the present invention. At the very least, and not as an attempt to limit the application of the doctrine of equivalents to the scope of the claims, each numerical parameter should at least be construed in light of the number of reported significant digits and by applying ordinary rounding techniques.

### Detailed Description

The present invention discloses the use of melt additive viscosity modifiers that modify the melt viscosity of aliphatic polyesters, such as polyhydroxyalknoate thermoplastics. The fine fibers are particularly useful for making absorbent or repellent aliphatic polyester nonwoven gowns and film laminate drapes used in surgery sterilization wraps for sterilization of equipment, as well as personal care absorbents such as feminine hygiene pads, diapers, incontinence pads, wipes, fluid filters, insulation and the like.

In one aspect, this invention provides fine fibers comprising a thermoplastic aliphatic polyester polymer, e.g., polylactic acid, polyhydroxybutyrate and the like, and one or more viscosity modifiers selected from the group of alkyl, alkenyl, aralkyl, or alkaryl carboxylates, or combinations thereof. The viscosity modifier is present in the melt extruded fiber in an amount sufficient to modify the melt viscosity of the aliphatic polyester. Typically, the viscosity modifier is present at less than 10 weight %, preferably less than 8 weight %, more preferably less than 7 %, more preferably less than 6 weight %, more preferably less than 3 weight %, and most preferably less than 2 % by weight based on the combined weight of the aliphatic polyester and viscosity modifier.

In another aspect, films, fabrics and webs constructed from the fine fibers are provided. The invention also provides useful articles made from fabrics and webs of fine fibers including medical drapes, sterilization wraps, medical gowns, aprons, filter media, industrial wipes and personal care and home care products such as diapers, facial tissue, facial wipes, wet wipes, dry wipes, disposable absorbent articles and garments such as disposable and reusable garments including infant diapers or training pants, adult incontinence products, feminine hygiene products such as sanitary napkins. panty liners and the like. The fine fibers of this invention also may be useful for producing thermal insulation for garments such as coats, jackets, gloves, cold weather pants, boots, and the like as well as acoustical insulation.

In yet another aspect, this invention provides multi-layer, aqueous liquid-absorbent articles comprising an aqueous media impervious backing sheet. For example, importantly some surgical drapes are liquid impervious to prevent liquid that is absorbed into the top sheet from wicking through to the skin surface where it would be contaminated with bacteria present on the skin. In other embodiments the construction may further comprise an aqueous media permeable topsheet, and an aqueous liquid-absorbent (i.e., hydrophilic) layer constructed of the above-described web or fabric juxtaposed there between useful, for instance, in constructing disposable diapers, wipes or towels, sanitary napkins, and incontinence pads.

In yet another aspect, a single or multi-layer aqueous repellent article such as a sterilization wrap, a surgical or medical gown or apron can be formed at least in part of a web of fine fibers described herein, and have aqueous fluid repellent properties. For example, an SMS web may be formed having fine fibers in at least the M (melt blown, blow microfiber) layer but they may also comprise the S (spunbond layer) as well. The M layer may have further incorporated a repellent additive at the surface of the fibers, such as a fluorochemical, silicone, hydrocarbon wax or combinations thereof. The repellent additive may be incorporated into the melt as the web is made, coated onto the fibers prior to web formation, or coated onto the formed or semi-formed web. In this manner, the sterilization wrap is rendered water repellent or the gown is rendered fluid repellent to avoid absorption of blood or other body fluids that may contain pathogenic microorganisms.

The fine fiber fabrics (nonwovens, wovens, or knits) of this invention may be rendered more repellent by treatment with numerous compounds. For example, the fabrics may be post web forming surface treatments which include paraffin waxes, fatty acids, bee's wax, silicones, fluorochemicals and combinations thereof. For example, the repellent finishes may be applied as disclosed in U.S. Patent Nos. 5,027,803; 6,960,642; and 7,199,197, all of which are incorporated by reference herein in its entirety. Repellent finishes may also be melt additives such as those described in U.S. Patent No. 6,262,180, which is incorporated by reference herein in its entirety.

This invention also provides a method of preparing the fine fibers from a mixture or blend of thermoplastic film-forming aliphatic polyester polymer, and at least one viscosity modifier. The viscosity modifier can be conveniently compounded with the resin in the hopper or elsewhere along the extruder as long as good mixing is achieved to render a substantially uniform mixture. Alternatively, the viscosity modifier may be added into the extruder directly (without precompounding), for example, using a positive displacement pump or weight loss feeder.

### POLYESTERS

Aliphatic polyesters useful in the present invention include homo- and copolymers of poly(hydroxyalkanoates), and homo- and copolymers of those aliphatic polyesters derived from the reaction product of one or more polyols with one or more polycarboxylic acids that is typically formed from the reaction product of one or more alkanediols with one or more alkanedicarboxylic acids (or acyl derivatives). Polyesters may further be derived from multifunctional polyols, e.g. glycerin, sorbitol, pentaerythritol, and combinations thereof, to form branched, star, and graft homo- and copolymers. Miscible and immiscible blends of aliphatic polyesters with one or more additional semicrystalline or amorphous polymers may also be used.

One useful class of aliphatic polyesters are poly(hydroxyalkanoates), derived by condensation or ring-opening polymerization of hydroxy acids, or derivatives thereof. Suitable poly(hydroxyalkanoates) may be represented by the formula:

H(O-R-C(O)-)ₙOH,

where R is an alkylene moiety that may be linear or branched having 1 to 20 carbon atoms, preferably 1 to 12 carbon atoms optionally substituted by catenary (bonded to carbon atoms in a carbon chain) oxygen atoms; n is a number such that the ester is polymeric, and is preferably a number such that the molecular weight of the aliphatic polyester is at least 10,000, preferably at least 30,000, and most preferably at least 50,000 daltons. Although higher molecular weight polymers generally yield films with better mechanical properties, for both melt processed and solvent cast polymers excessive viscosity is typically undesirable. The molecular weight of the aliphatic polyester is typically less than 1,000,000, preferably less than 500,000, and most preferably less than 300,000 daltons. R may further comprise one or more caternary (i.e. in chain) ether oxygen atoms. Generally, the R group of the hydroxy acid is such that the pendant hydroxyl group is a primary or secondary hydroxyl group.

Useful poly(hydroxyalkanoates) include, for example, homo- and copolymers of poly(3-hydroxybutyrate), poly(4-hydroxybutyrate), poly(3-hydroxyvalerate), poly(lactic acid) (as known as polylactide), poly(3-hydroxypropanoate), poly(4-hydropentanoate), poly(3-hydroxypentanoate), poly(3-hydroxyhexanoate), poly(3-hydroxyheptanoate), poly(3-hydroxyoctanoate), polydioxanone, polycaprolactone, and polyglycolic acid (i.e. polyglycolide). Copolymers of two or more of the above hydroxy acids may also be used, for example, poly(3-hydroxybutyrate-co-3-hydroxyvalerate), poly(lactate-co-3-hydroxypropanoate), poly(glycolide-co-p-dioxanone), and poly(lactic acid-co-glycolic acid). Blends of two or more of the poly(hydroxyalkanoates) may also be used, as well as blends with one or more polymers and/or copolymers.

The aliphatic polyester may be a block copolymer of poly(lactic acid-co-glycolic acid). Aliphatic polyesters useful in the inventive fine fibers may include homopolymers, random copolymers, block copolymers, star-branched random copolymers, star-branched block copolymers, dendritic copolymers, hyperbranched copolymers, graft copolymers, and combinations thereof.

Another useful class of aliphatic polyesters includes those aliphatic polyesters derived from the reaction product of one or more alkanediols with one or more alkanedicarboxylic acids (or acyl derivatives). Such polyesters have the general formula: 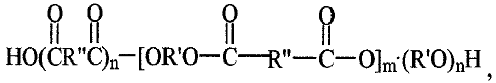 where R' and R" each represent an alkylene moiety that may be linear or branched having from 1 to 20 carbon atoms, preferably 1 to 12 carbon atoms, and m is a number such that the ester is polymeric, and is preferably a number such that the molecular weight of the aliphatic polyester is at least 10,000, preferably at least 30,000, and most preferably at least 50,000 daltons, but less than 1,000,000, preferably less than 500,000 and most preferably less than 300,000 daltons. Each n is independently 0 or 1. R' and R" may further comprise one or more caternary (i.e. in chain) ether oxygen atoms.

Examples of aliphatic polyesters include those homo-and copolymers derived from (a) one or more of the following diacids (or derivative thereof): succinic acid, adipic acid, 1,12 dicarboxydodecane, fumaric acid, glutartic acid, diglycolic acid, and maleic acid; and (b) one of more of the following diols: ethylene glycol, polyethylene glycol, 1,2-propane diol, 1,3-propanediol, 1,2-propanediol, 1,2-butanediol, 1,3-butanediol, 1,4-butanediol, 2,3-butanediol, 1,6-hexanediol, 1,2 alkane diols having 5 to 12 carbon atoms, diethylene glycol, polyethylene glycols having a molecular weight of 300 to 10,000 daltons, preferably 400 to 8,000 daltons, propylene glycols having a molecular weight of 300 to 4000 daltons, block or random copolymers derived from ethylene oxide, propylene oxide, or butylene oxide, dipropylene glycol and polypropylene glycol, and (c) optionally a small amount, i.e. 0.5-7.0 mole% of a polyol with a functionality greater than two such as glycerol, neopentyl glycol, and pentaerythritol.

Such polymers may include polybutylenesuccinate homopolymer, polybutylene adipate homopolymer, polybutyleneadipate-succinate copolymer, polyethylenesuccinate-adipate copolymer, polyethylene glycol succinate and polyethylene adipate homopolymer.

Commercially available aliphatic polyesters include poly(lactide), poly(glycolide), poly(lactide-co-glycolide), poly(L-lactide-co-trimethylene carbonate), poly(dioxanone), poly(butylene succinate), and poly(butylene adipate).

Useful aliphatic polyesters include those derived from semicrystalline polylactic acid. Poly(lactic acid) or polylactide has lactic acid as its principle degradation product, which is commonly found in nature, is non-toxic and is widely used in the food, pharmaceutical and medical industries. The polymer may be prepared by ring-opening polymerization of the lactic acid dimer, lactide. Lactic acid is optically active and the dimer appears in four different forms: L,L-lactide, D,D-lactide, D,L-lactide (meso lactide) and a racemic mixture of L,L- and D,D-. By polymerizing these lactides as pure compounds or as blends, poly(lactide) polymers may be obtained having different stereochemistries and different physical properties, including crystallinity. The L,L- or D,D-lactide yields semicrystalline poly(lactide), while the poly(lactide) derived from the D,L-lactide is amorphous.

The polylactide preferably has a high enantiomeric ratio to maximize the intrinsic crystallinity of the polymer. The degree of crystallinity of a poly(lactic acid) is based on the regularity of the polymer backbone and the ability to crystallize with other polymer chains. If relatively small amounts of one enantiomer (such as D-) is copolymerized with the opposite enantiomer (such as L-) the polymer chain becomes irregularly shaped, and becomes less crystalline. For these reasons, when crystallinity is favored, it is desirable to have a poly(lactic acid) that is at least 85% of one isomer, more preferably at least 90% of one isomer, or even more preferably at least 95% of one isomer in order to maximize the crystallinity.

An approximately equimolar blend of D-polylactide and L-polylactide is also useful. This blend forms a unique crystal structure having a higher melting point (∼210°C) than does either the D-poly(lactide) and L-(polylactide) alone (∼160°C), and has improved thermal stability. See H. Tsuji et. al., Polymer, 40 (1999) 6699-6708.

Copolymers, including block and random copolymers, of poly(lactic acid) with other aliphatic polyesters may also be used. Useful co-monomers include glycolide, beta-propiolactone, tetramethylglycolide, beta-butyrolactone, gamma-butyrolactone, pivalolactone, 2-hydroxybutyric acid, alpha-hydroxyisobutyric acid, alpha-hydroxyvaleric acid, alpha-hydroxyisovaleric acid, alpha-hydroxycaproic acid, alpha-hydroxyethylbutyric acid, alpha-hydroxyisocaproic acid, alpha-hydroxy-beta-methylvaleric acid, alpha-hydroxyoctanoic acid, alpha-hydroxydecanoic acid, alpha-hydroxymyristic acid, and alpha-hydroxystearic acid.

Blends of poly(lactic acid) and one or more other aliphatic polyesters, or one or more other polymers may also be used. Examples of useful blends include poly(lactic acid) and poly(vinyl alcohol), polyethylene glycol/polysuccinate, polyethylene oxide, polycaprolactone and polyglycolide.

Poly(lactide)s may be prepared as described in U.S. Patent Nos. 6,111,060 (Gruber, et al.), 5,997,568 (Liu), 4,744,365 (Kaplan et al.), 5,475,063 (Kaplan et al.), 6,143,863 (Gruber et al.), 6,093,792 (Gross et al.), 6,075,118 (Wang et al.), and 5,952,433 (Wang et al.),WO 98/24951 (Tsai et al.), WO 00/12606 (Tsai et al.), WO 84/04311 (Lin), U.S. 6,117,928 (Hiltunen et al.), U.S. 5,883,199 (McCarthy et al.), WO 99/50345 (Kolstad et al.), WO 99/06456 (Wang et al.), WO 94/07949 (Gruber et al.), WO 96/22330 (Randall et al.), and WO 98/50611 (Ryan et al.), the disclosure of each incorporated herein by reference. Reference may also be made to J.W. Leenslag, et al., J. Appl. Polymer Science, vol. 29 (1984), pp 2829-2842, and H.R. Kricheldorf, Chemosphere, vol. 43, (2001) 49-54.

The molecular weight of the polymer should be chosen so that the polymer may be processed as a melt. For polylactide, for example, the molecular weight may be from about 10,000 to 1,000,000 daltons, and is preferably from about 30,000 to 300,000 daltons. By "melt-processible", it is meant that the aliphatic polyesters are fluid or can be pumped or extruded at the temperatures used to process the articles (e.g. make the fine fibers in BMF), and do not degrade or gel at those temperatures to the extent that the physical properties are so poor as to be unusable for the intended application. Thus, many of the materials can be made into nonwovens using melt processes such as spun bond, blown microfiber, and the like. Certain embodiments also may be injection molded. The aliphatic polyester may be blended with other polymers but typically comprises at least 50 weight percent, preferably at least 60 weight percent, and most preferably at least 65 weight percent of the fine fibers.

### VISCOSITY MODIFIER

The fine fibers disclosed herein include one or more viscosity modifiers to reduce the average diameter of the fiber during the melt process (e.g. blown microfiber (BMF), spunbond, or injection molding). We have found that the addition of most known plasticizers for the aliphatic polyester thermoplastics result in a very gradual viscosity reduction. This is generally not useful for producing fine fibers of sufficient mechanical strength since the plasticizers degrade polymer strength. Viscosity reduction can be detected in the extrusion/BMF equipment by recording the pressures within the equipment.

The viscosity modifiers of the present invention result in a dramatic viscosity reduction, and thus reduce back pressure during extrusion or thermal processing. In many cases, the viscosity reduction is so great that the melt processing temperature must be reduced in order to maintain sufficient melt strength. Often the melt temperature is reduced by 30°C or more.

In applications in which biodegradability is important, it may be desirable to incorporate biodegradable viscosity modifiers, which typically include ester and/or amide groups that may be hydrolytically or enzymatically cleaved. The viscosity modifiers useful in the fine fibers described herein include viscosity modifiers with the following structure:

R-CO₂⁻M⁺

Where R is alkyl or alkylene of C8-C30, which is branched or straight chain, or C12-C30 aralkyl, and may be optionally substituted with 0-100 alkylene oxide groups such as ethylene oxide, propylene oxide groups, oligameric lactic and/or glycolic acid or a combination thereof;
M is H, an alkali metal or an alkaline earth metal salt, preferably Na+, K+, or Ca++, or amine salts including tertiary and quaternary amines such as protonated triethanolamine, tetramethylammonium and the like.

In the formula above, the ethylene oxide groups and propylene oxide groups can occur in reverse order as well as in a random, sequential, or block arrangement.

In certain preferred embodiments, the viscosity modifiers useful to form fine fibers are selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof. The carboxylic acid equivalents of the carboxylates may also function as viscosity modifiers. Combinations of various viscosity modifiers can also be used. As used herein a lactylate is a surfactant having a hydrophobe and a hydrophile wherein the hydrophile is at least in part an oligamer of lactic acid having 1-5 lactic acid units, and typically having 1-3 lactic acid units. A preferred lactylate is calcium stearoyl lactylate from Rita Corp. which is reported to have the following structure: [CH₃(CH₂)₁₆C(O)O-CH(CH₃)-C(O)O- CH(CH3)-C(O)O-]₂ Ca⁺⁺ ALkyl lactylates are a preferred class of viscosity modifiers since these also are made from resource renewable materials.

The viscosity modifiers typically melt at or below the extrusion temperature of the thermoplastic aliphatic polyester composition. This greatly facilitates dispersing or dissolving the viscosity modifier in the polymer composition. Mixtures of viscosity modifiers may be employed to modify the melting point. For example, mixtures of alkyl carboxylates may be preformed or an alkyl carboxylate may be blended with a nonionic surfactant such as a polyethoxylated surfactant. The necessary processing temperature may be altered by addition of nonsurfactant components as well such as plasticizers for the thermoplastics aliphatic polyester. For example, when added to polylactic acid compositions, the viscosity modifiers preferably have a melting point of less than 200°C, preferably less than 180°C, more preferably less than 170°C, and even more preferably less than 160°C.

In some embodiments, when used in the fine fibers, the viscosity modifiers are present in a total amount of at least 0.25 wt-%, at least 0.5 wt-%, at least 1.0 wt-%, or at least 2.0 wt-%, based on the total weight of the fine fibers. In certain embodiments, in which a very low viscosity melt is desired and/or a low melt temperature is preferred, the fine fibers comprise greater than 2 wt. %, greater than 3 wt. %, or even greater than 5 wt. % of the viscosity modifier based on the weight of the aliphatic polyester polymer in the fine fibers.

For melt processing, preferred viscosity modifiers have low volatility and do not decompose appreciably under process conditions. The preferred viscosity modifiers contain less than 10 wt. % water, preferably less than 5% water, and more preferably less than 2 wt. % and even more preferably less than 1% water (determined by Karl Fischer analysis). Moisture content is kept low in order to prevent hydrolysis of the aliphatic polyester or other hydrolytically sensitive compounds in the fine fibers.

The viscosity modifiers may be carried in a nonvolatile carrier. Importantly, the carrier is typically thermally stable and can resist chemical breakdown at processing temperatures which may be as high as 150°C, 200°C, 250°C, or even as high as 300°C. Preferred carriers for hydrophilic articles include polyalkylene oxides such as polyethylene glycol, polypropylene glycol, random and block copolymers of ethylene oxide and propylene oxide, thermally stable polyhydric alcohols such as propylene glycol, glycerin, polyglycerin, and the like. The polyalkylene oxides/polyalkylene glycols may be linear or branched depending on the initiating polyol. For example, a polyethylene glycol initiated using ethylene glycol would be linear but one initiated with glycerin, trimethylolpropane, or pentaerythritol would be branched.

### OPTIONAL COMPONENTS

Other optional components may be included in the fine fibers, or the articles made therefrom, as described herein.

An antimicrobial component may be added to impart antimicrobial activity to the fine fibers. The antimicrobial component is the component that provides at least part of the antimicrobial activity, i.e., it has at least some antimicrobial activity for at least one microorganism. It is preferably present in a large enough quantity to be released from the fine fibers and kill bacteria. It may also be biodegradable and/or made or derived from renewable resources such as plants or plant products. Biodegradable antimicrobial components can include at least one functional linkage such as an ester or amide linkage that can be hydrolytically or enzymatically degraded.

Examples of antimicrobial components suitable for use in the present invention include those described in Applicants' co-pending application, U.S. Patent Application Publication No. 2008-0142023-A1, and incorporated by reference herein in its entirety.

Certain antimicrobial components are uncharged and have an alkyl or alkenyl hydrocarbon chain containing at least 7 carbon atoms. For melt processing, preferred antimicrobial components have low volatility and do not decompose under process conditions. The preferred antimicrobial components contain less than 2 wt. % water, and more preferably less than 0.10 wt. % (determined by Karl Fischer analysis). Moisture content is kept low in order to prevent hydrolysis of the aliphatic polyester during extrusion.

When used, the antimicrobial component content (as it is ready to use) is typically at least 1 wt. %, 2 wt. %, 5 wt. %, 10 wt. % and sometimes greater than 15 wt. %. In certain embodiments, for example applications in which a low strength is desired, the antimicrobial component comprises greater than 20 wt. %, greater than 25 wt. %, or even greater than 30 wt. % of the fine fibers.

Certain antimicrobial components are amphiphiles and may be surface active. For example, certain antimicrobial alkyl monoglycerides are surface active. For certain embodiments of the invention that include antimicrobial components, the antimicrobial component is considered distinct from a viscosity modifier component.

The fine fibers may further comprise organic and inorganic fillers. For implantable applications biodegradable, resorbable, or bioerodible inorganic fillers may be particularly appealing. These materials may help to control the degradation rate of the polymer fine fibers. For example, many calcium salts and phosphate salts may be suitable. Exemplary biocompatible resorbable fillers include calcium carbonate, calcium sulfate, calcium phosphate, calcium sodium phosphates, calcium potassium phosphates, tetracalcium phosphate, alpha.-tricalcium phosphate, beta-tricalcium phosphate, calcium phosphate apatite, octacalcium phosphate, dicalcium phosphate, calcium carbonate, calcium oxide, calcium hydroxide, calcium sulfate dihydrate, calcium sulfate hemihydrate, calcium fluoride, calcium citrate, magnesium oxide, and magnesium hydroxide. A particularly suitable filler is tribasic calcium phosphate (hydroxy apatite).

The fine fibers may also comprise anionic surfactants that impart durable hydrophilicity. Examples of anionic surfactants suitable for use in the present invention include those described in Applicants' co-pending applications, U.S. Serial No. 61/061,088, filed June 12, 2008, and PCT Application No.__________, citing priority to the foregoing and filed on June 11, 2009, incorporated by reference herein in its entirety.

Plasticizers may be used with the aliphatic polyester thermoplastic and include, for example, glycols such glycerin; propylene glycol, polyethoxylated phenols, mono or polysubstituted polyethylene glycols, higher alkyl substituted N-alkyl pyrrolidones, sulfonamides, triglycerides, citrate esters, esters of tartaric acid, benzoate esters, polyethylene glycols and ethylene oxide propylene oxide random and block copolymers having a molecular weight less than 10,000 daltons preferably less than about 5000 daltons, more preferably less than about 2500 daltons; and combinations thereof.

Other additional components include antioxidants, colorants such as dyes and/or pigments, antistatic agents, fluorescent brightening agents, odor control agents, perfumes and fragrances, active ingredients to promote wound healing or other dermatological activity, combinations thereof, and the like.

### APPLICATIONS

Articles that may be made of fine fibers may include medical drapes and gowns, including surgical drapes, procedural drapes, plastic specialty drapes, incise drapes, barrier drapes, barrier gowns, SMS gowns, and the like; sterilization wraps; wound dressings, wound absorbents, and wound contact layers; surgical sponges use to absorb blood and body fluids during surgery; surgical implants; and other medical devices. Articles made of the fine fibers may be solvent, heat, or ultrasonically welded together as well as being welded to other compatible articles. The fine fibers may be used in conjunction with other materials to form constructions such as sheath/core materials, laminates, compound structures of two or more materials, or useful as coatings on various medical devices. The fine fibers described herein may be useful in the fabrication of surgical sponges.

In certain embodiments, the fine fiber web is a component of a surgical drape. As used herein a "surgical drape" is a textile that is used to cover the patient and/or instrumentation and other objects during invasive procedures such as surgery. The drapes are most often provided sterile. The fine fiber webs of this invention can be sterilized by conventional methods such as sterilizing gases including steam, ethylene oxide, hydrogen peroxide and the like. A significant advantage is that the fine fiber webs can be sterilized by gamma irradiation without significant loss in physical properties.

The purpose of the drape is to provide a sterile surface and to contain microbial contamination from the patient and/or equipment. Thus, the fine fiber web may be coated with an impervious film. Any suitable film can be used. When laminated to an impervious film the fine fiber web rendered hydrophilic as described in Applicants' co-pending application, U.S. Serial No. 61/061,088, filed June 12, 2008, and PCT Application No.________, citing priority to the foregoing and filed on June 11, 2009, incorporated by reference herein in its entirety; and constructed as described in co-pending application, U.S. Serial No. 61/165,316, filed March 31, 2009 and U.S. Serial No.__________ , citing priority to the foregoing application and filed on the same date herewith (Attorney Docket No. 64410US005), each incorporated by reference in their entirety. In this manner, the drape is absorbent and still a barrier. Alternatively, the drape may be constructed of a fine fiber-containing web which has been treated with a repellent additive as described above.

In certain embodiments the fine fiber web is a component of a surgical gown. As used herein a "surgical gown" is a textile that is used to cover the clinician during invasive procedures such as surgery. Additionally, the gowns may be used for many other procedures where the clinician wishes to protect themselves from contamination. The gowns are most often provided sterile and may be sterilized as described above for the surgical drapes. Typically the gown is constructed of a fine fiber-containing web which has been treated with the repellent additive as described above. The purpose of the gown is to provide a sterile surface and to contain microbial contamination from the clinician so that it does not contaminate a sterile field. Importantly, the gowns also may be used to protect the clinician from exposure to infectious agents such as bacteria, spores, virus, mycobacterium etc. Thus, the fine fiber web may be coated with an impervious film. Any suitable film can be used. Preferably, if a film is used it is microporous to allow moisture evaporation. Alternatively, the gown may be constructed of a fine fiber containing web which has been treated to be repellent additive as described above. In this manner, any blood or body fluids that contact the gown are repelled and will not soak in to contact the clinician.

The preferred hydrophilic additive surfactants of the present invention allow for adhesive, thermal, and/or ultrasonic bonding of fabrics and films made thereof. The fine fibers are particularly suitable for use in surgical drapes and gowns. Non-woven web and sheets comprising the fine fibers can be heat sealed to form strong bonds allowing specialty drape fabrication; can be made from renewable resources which can be important in disposable products; and can have high surface energy to allow wettability and fluid absorbency in the case of non-wovens. In other applications, a low surface energy may be desirable to impart fluid repellency.

It is believed that such non-woven materials can be sterilized by gamma radiation or electron beam without significant loss of physical strength (tensile strength for a 1 mil thick film does not decrease by more than 20% and preferably by not more than 10% after exposure to 2.5 Mrad gamma radiation from a cobalt gamma radiation source and aged at 23 °- 25°C for 7 days.

The hydrophilic characteristic of the fine fibers may improve articles such as wound and surgical dressings by improving absorbency. If the fine fibers is used in a wound dressing backing film, the film may be partially (e.g. zone or pattern) coated or completely coated with various adhesives, including but not limited to pressure sensitive adhesives (PSAs), such as acrylic and block copolymer adhesives, hydrogel adhesives, hydrocolloid adhesives, and foamed adhesives. PSAs can have a relatively high moisture vapor transmission rate to allow for moisture evaporation. Suitable pressure sensitive adhesives include those based on acrylates, polyurethanes, KRATON and other block copolymers, silicones, rubber based adhesives as well as combinations of these adhesives. The preferred PSAs are medical adhesives that are applied to skin such as the acrylate copolymers described in U.S. Patent No. RE 24,906, the disclosure of which is hereby incorporated by reference, particularly a 97:3 iso-octyl acrylate:acrylamide copolymer. Also preferred is an 70:15:15 iso-octyl acrylate-ethyleneoxide acrylate:acrylic acid terpolymer, as described in U.S. Patent No. 4,737,410 (Example 31), the disclosure of which is hereby incorporated by reference. Other useful adhesives are described in U.S. Patent Nos. 3,389,827; 4,112,213; 4,310,509; and 4,323,557; the disclosures of which are hereby incorporated by reference. Inclusion of medicaments or antimicrobial agents in the adhesive is also contemplated, as described in U.S. Patent Nos. 4,310,509 and 4,323,557.

Other medical devices that may be made, in whole or in part, of the fine fibers include: sutures, suture fasteners, surgical mesh, slings, orthopedic pins (including bone filling augmentation material), adhesion barriers, stents, guided tissue repair/regeneration devices, articular cartilage repair devices, nerve guides, tendon repair devices, atrial septal defect repair devices, pericardial patches, bulking and filling agents, vein valves, bone marrow scaffolds, meniscus regeneration devices, ligament and tendon grafts, ocular cell implants, spinal fusion cages, skin substitutes, dural substitutes, bone graft substitutes, bone dowels, and hemostats.

The fine fibers of the present invention may also be useful in consumer hygiene products, such as adult incontinence, infant diapers, feminine hygiene products, and others as described in Applicants' co-pending application, U.S. Patent Application Publication No. 2008-0200890-A1, and incorporated by reference herein in its entirety.

In certain embodiments, a wrap may be formed that is used to wrap clean instruments prior to surgery or other procedure requiring sterile tools. These wraps allow penetration of sterilizing gasses such as steam, ethylene oxide, hydrogen peroxide, etc. but they do not allow penetration of bacteria. The wraps may be made into a single or multi-layer water repellent article which can be formed at least in part of a web of fine fibers described herein and having aqueous fluid repellent properties. For example, a SMS, SMMS, or other nonwoven construction web may be formed having fine fibers in at least the M (melt blown, blown microfiber) layer but they may also comprise the S (spunbond layer as well). The M layer may have further incorporated therein or thereon a repellent additive such as a fluorochemical. Suitable fluorochemicals and silicones that serve as repellent additives are described below.

A sterilization wrap constructed from such a single or multi-layer repellent article described herein possesses all of the properties required of a sterilization wrap; i.e., permeability to steam or ethylene oxide or other gaseous sterilant during sterilization (and during drying or aeration) of the articles it encloses, repellency of liquid water during storage to avoid contamination of the contents of the wrap by water-borne contaminants, and a tortuous path barrier to contamination by air- or water-borne microbes during storage of the sterilized pack.

### REPELLENT ADDITIVE

Preferred fluorochemicals comprise a perfluoroalkyl group having at least 4 carbon atoms. These fluorochemicals may be small molecules, oligamers, or polymers. Silicone fluid repellents also may be suitable. In some instances hydrocarbon-type repellents may also be suitable.

Classes of fluorochemical agents or compositions useful in this invention include compounds and polymers containing one or more fluoroaliphatic radicals, R_{f}. In general, fluorochemical agents or compositions useful as a repellent additive comprise fluorochemical compounds or polymers containing fluoroaliphatic radicals or groups, R_{f}. The fluoroaliphatic radical, R_{f}, is a fluorinated, stable, inert, non-polar, preferably saturated, monovalent moiety which is both hydrophobic and oleophobic. It can be straight chain, branched chain, or, if sufficiently large, cyclic, or combinations thereof, such as alkylcycloaliphatic radicals. The skeletal chain in the fluoroaliphatic radical can include catenary divalent oxygen atoms and/or trivalent nitrogen atoms bonded only to carbon atoms. Generally R_{f} will have 3 to 20 carbon atoms, preferably 6 to about 12 carbon atoms, and will contain about 40 to 78 weight percent, preferably 50 to 78 weight percent, carbon-bound fluorine. The terminal portion of the R_{f} group has at least one trifluoromethyl group, and preferably has a terminal group of at least three fully fluorinated carbon atoms, e.g., CF₃CF₂CF₂--. The preferred R_{f} groups are fully or substantially fluorinated, as in the case where R_{f} is perfluroalkyl, CₙF₂ₙ₊₁--,

Examples of such compounds include, for example, fluorochemical urethanes, ureas, esters, amines (and salts thereof), amides, acids (and salts thereof), carbodiimides, guanidines, allophanates, biurets, and compounds containing two or more of these groups, as well as blends of these compounds.

Useful fluorochemical polymers containing R_{f} radicals include copolymers of fluorochemical acrylate and/or methacrylate monomers with co-polymerizable monomers, including fluorine-containing and fluorine-free monomers, such as methyl methacrylate, butyl acrylate, octadecyl methacrylate, acrylate and methacrylate esters of poly(oxyalkylene) polyol oligomers and polymers, e.g., poly(oxyethylene) glycol dimethacrylate, glycidyl methacrylate, ethylene, vinyl acetate, vinyl chloride, vinylidene chloride, vinylidene fluoride, acrylonitrile, vinyl chloroacetate, isoprene, chloroprene, styrene, butadiene, vinylpyridine, vinyl alkyl esters, vinyl alkyl ketones, acrylic and methacrylic acid, 2-hydroxyethyl acrylate, N-methylolacrylamide, 2-(N,N,N-trimethylammonium)ethyl methacrylate and the like.

The relative amounts of various comonomers which can be used with the fluorochemical monomer will generally be selected empirically, and will depend on the substrate to be treated, the properties desire from the fluorochemical treatment, i.e., the degree of oil and/or water repellency desired, and the mode of application to the substrate.

Useful fluorochemical agents or compositions include blends of the various classes of fluorochemical compounds and/or polymers described above. Also, blends of these fluorochemical compounds or polymers with fluorine-free compounds, e.g., N-acyl aziridines, or fluorine-free polymers, e.g., polyacrylates such as poly(methyl methacrylate) and poly(methyl methacrylate-co-decyl acrylate), polysiloxanes and the like.

The fluorochemical agents or compositions can include non-interfering adjuvants such as wetting agents, emulsifiers, solvents (aqueous and organic), dyes, biocides, fillers, catalysts, curing agents and the like. The final fluorochemical agent or composition should contain, on a solids basis, at least about 5 weight percent, preferably at least about 10 weight percent carbon-bound fluorine in the form of said R_{f} groups in order to impart the benefits described in this invention. Such fluorochemicals are generally known and commercially available as perfluoroaliphatic group bearing water/oil repellent agents which contain at least 5 percent by weight of fluorine, preferably 7 to 12 percent of fluorine in the available formulations.

By the reaction of the perfluoroaliphatic thioglycols with diisocyanates, there results perfluoroaliphatic group-bearing polyurethanes. These products are normally applied in aqueous dispersion for fiber treatment. Such reaction products are described in U.S. Patent No. 4,054,592, incorporated herein by reference.

Another group of suitable compounds are perfluoroaliphatic group-bearing N-methylol condensation products. These compounds are described in U.S. Patent No. 4,477,498, incorporated herein by reference where the emulsification of such products is dealt with in detail.

The perfluoroaliphatic group-bearing polycarbodimides are, e.g., obtained by reaction of perfluoroaliphatic sulfonamide alkanols with polyisocyanates in the presence of suitable catalysts. This class of compounds can be used by itself, but often is used with other Regroup bearing compounds, especially with (co)polymers. Thus, another group of compounds which can be used in dispersions is mentioned. Among these compounds all known polymers bearing fluoroaliphatic residues can be used, also condensation polymers, such as polyesters and polyamides which contain the corresponding perfluoroaliphatic groups, are considered but especially (co)polymers on the basis of e.g. R_{f} -acrylates and R_{f} -methacrylates, which can contain different fluorine-free vinyl compounds as comonomers. In DE-A 2 310 801, these compounds are discussed in detail. The manufacture of R_{f} -group bearing polycarbodimides as well as the combination of these compounds with each other is also described in detail.

Besides the aforementioned perfluoroaliphatic group-bearing agents, further fluorochemical components may be used, for example, R_{f} -group-bearing guanidines, U.S. Patent No. 4,540,479, R_{f} -group-bearing allophanates, U.S. Patent No. 4,606,737 and R_{f} -group-bearing biurets, U.S. Patent No. 4,668,406, the disclosures which are incorporated herein by reference. These classes are mostly used in combination. Others include fluoroalkyl-substituted siloxanes, e.g., CF₃(CF₂)₆CH₂O(CH₂)₃Si(OC₂H₅)₃- .

The useful compounds show, in general, one or more perfluoroaliphatic residues with preferably at least 4 carbon atoms, especially 4 to 14 atoms each. An exemplary fluorochemical is a formulation of 70% solvents and 30% emulsified solid fluorochemical polymers. The formulation includes as solvents 11% methyl isobutyl ketone, 6% ethylene glycol and 53% water. The fluorochemical polymers are a 50/50 blend of 5/95 copolymer of butyl acrylate and C₈F₁₇SO₂(CH₃)C₂H₄O-CCH=CH₂ prepared as described in U.S. Patent No. 3,816,229, incorporated herein by reference (see especially column 3, lines 66-68 and column 4, lines 1-11) for a 10/90 copolymer. The second component of the 50/50 blend is a copolymer prepared from 1 mole of a trifunctional phenyl isocyanate (available from Upjohn Company under the name PAPI), 2 moles of C₈F₁₇N(CH₂CH₃)CH₂CH₂OH and 1 mole of stearyl alcohol prepared as described in U.S. Patent No. 4,401,780, incorporated herein by reference (see especially Table 1, C₂ under footnote A). Emulsifiers used are conventional commercially available materials such as polyethoxylated quaternary ammonium compounds (available under the name 5% Ethoquad 18/25 from Akzo Chemie America) and 7.5% of a 50/50 mixture of C₈F₁₇SO₂NHC₃H₆N(CH₃)₃Cl and a polyethoxylated sorbitan monooleate (available from ICI Limited under the name TWEEN 80). Such fluorochemicals are non-yellowing and particularly non-irritating to the skin as well as providing articles that are stable having excellent long term aging properties. Exemplary fluorochemicals are available under the trade designations SCOTCHGARD, SCOTCH-RELEASE, and 3M BRAND TEXTILE CHEMICAL and are commercially from the 3M Company. Other commercially available materials include materials that use fluorotelomer chemistry materials provided by DuPont (available from duPont deNemours and Company, Wilmington, Del.).

Suitable silicones for use to obtain the low surface energy layers of the instant invention include any of the silicones known to those skilled in the art to provide water repellency and optionally oil repellency to fibers and films. Silicone fluids typically consist of linear polymers of rather low molecular weight, namely about 4000-25,000. Most commonly the polymers are polydimethylsiloxanes.

For use as fluids with enhanced thermal stability, silicones containing both methyl and phenyl groups are often used. Generally, the phenyl groups make up 10-45% of the total number of substituent groups present. Such silicones are generally obtained by hydrolysis of mixtures of methyl- and phenylchlorosilanes. Fluids for use in textile treatment may incorporate reactive groups so that they may be cross-linked to give a permanent finish. Commonly, these fluids contain Si--H bonds (introduced by including methyldichlorosilane in the polymerization system) and cross-linking occurs on heating with alkali.

Examples of suitable silicones are those available from Dow-Coming Corporation such as C2-0563 and from General Electric Corporation such as GE-SS4098. Especially preferred silicone finishes are disclosed in U.S. Patent No. 5,045,387.

### METHODS OF MANUFACTURING

Articles comprising the fine fibers may be made by processes known in the art for making products like polymer sheets from polymer resins. For many applications, such articles can be placed in water at 23°C without substantial loss of physical integrity (e.g. tensile strength) after being immersed 2 hours and dried. Typically, these articles contain little or no water. The water content in the article after extruding, injection molding or solvent casting is typically less than 10% by weight, preferably less than 5% by weight, more preferably less than 1% by weight and most preferably less than 0.2% by weight.

As part of the process for making the fine fibers, the aliphatic polyester in a melt form is mixed in a sufficient amount relative to the viscosity modifier to yield fine fibers having average diameter characteristics as described herein.

A variety of equipment and techniques are known in the art for melt processing polymeric fine fibers. Such equipment and techniques are disclosed, for example, in U.S. Patent No. 3,565,985 (Schrenk et al.); U.S. Patent No. 5,427,842 (Bland et. al.); U.S. Patent Nos. 5,589,122 and 5,599,602 (Leonard); and U.S. Patent No. 5,660,922 (Henidge et al.). Examples of melt processing equipment include, but are not limited to, extruders (single and twin screw), Banbury mixers, and Brabender extruders for melt processing the inventive fine fibers.

The ingredients of the fine fibers may be mixed in and conveyed through an extruder to yield a polymer, preferably without substantial polymer degradation or uncontrolled side reactions in the melt.. Potential degradation reactions include transesterification, hydrolysis, chain scission and radical chain define fibers, and process conditions should minimize such reactions. The processing temperature is sufficient to mix the biodegradable aliphatic polyester viscosity modifier, and allow extruding the polymer.

The (BMF) meltblowing process is a method of forming a nonwoven fiber web where a polymer fluid, either melt or solution, is extruded through one or more rows of holes then impinged by a high velocity gas jet. The gas jet, typically heated air, entrains and draws the polymer fluid and helps to solidify the polymer into a fiber. The solid fiber is then collected on solid or porous surface as a nonwoven web. This process is described by Van Wente in "Superfine Thermoplastic Fibers", Industrial Engineering Chemistry, vol. 48, pp. 1342-1346. An improved version of the meltblowing process is described by Buntin et al. as described in U.S. Patent No. 3,849,241, and incorporated by reference herein in its entirety.

The viscosity modifiers described herein need not be added to the fiber extrusion process in a pure state. The viscosity modifiers may be compounded with the aliphatic polyester, or other materials prior to extrusion. Commonly, when additives such as viscosity modifiers are compounded prior to extrusion, they are compounded at a higher concentration than desired for the final fiber. This high concentration compound is referred to as a masterbatch. When a masterbatch is used, the masterbatch will generally be diluted with pure polymer prior to entering the fiber extrusion process. Multiple additives may be present in a masterbatch, and multiple masterbatches may be used in the fiber extrusion process.

An alternative melt blown process that may benefit from the use of viscosity modifiers as provided herein is described in U.S. Patent Application Publication No. 2008-0160861-A1, and incorporated by reference herein in its entirety.

The fine fiber webs may additionally be manufactured by the process as described in co-pending application, U. Serial No. 61/165,316, filed March 31, 2009 and U.S. Serial No.________________, citing priority to the foregoing application and filed on the same date herewith (Attorney Docket No. 64410US005), each incorporated by reference in their entirety.

The invention will be further clarified by the following examples which are exemplary and not intended to limit the scope of the invention.

### TEST METHODS

### Effective Fiber Diameter

Fiber diameter is measured using the Effective Fiber Diameter (EFD) method developed by Davies using basis weight, web thickness, and pressure drop to estimate the average fiber diameter of a fiber web. Davies, C.N., The Reparation of Airborne Dust and Particles, Inst. of Mech. Engineers, London, Proceedings 1B, 1952.

Average fiber diameter can be measured in several ways including microscopy, laser diffraction, and fluid flow resistance. Davies (Davies, C.N., The Separation of Dust and Particles, Inst. of Mech. Engineers, London, Proceedings 1B, 1952) developed a correlation for determining the average diameter of a fiber web using the air flow resistance, web thickness, and web basis weight. Air flow resistance was measured by recording the pressure drop of a 11.4 centimeter diameter web sample at an air flow rate of 32 liters per minute. Web thickness was measured on a 13.3 centimeter diameter circular web sample with an applied pressure of 150 Pa. Web basis weight was measured by weighing a 13.3 inch diameter web sample. The equations described by Davies were then used to determine the effective fiber diameter (EFD) of the web, expressed in units of microns (1 micron = 10E-6 meters).

### Shrinkage

After extrusion, the fine fiber webs were also measured for shrinkage by placing 10cm x 10cm squares of the web on aluminum trays in an oven at 80°C for approximately 14 hours. After aging the squares were measured and the average linear shrinkage was recorded.

The invention is summarized by the following items:
1. A fine fiber, comprising
   one or more thermoplastic aliphatic polyesters; and
   a viscosity modifier selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof.
2. The fine fiber of item 1, wherein the aliphatic polyester is selected from the group consisting of one or more poly(lactic acid), poly(glycolic acid), poly(lactic-co-glycolic acid), polybutylene succinate, polyhydroxybutyrate, polyhydroxyvalerate, blends, and copolymers thereof.
3. The fine fiber of item 1, wherein the viscosity modifier has the following structure:

   R-CO₂⁻M⁺

   Wherein R is an alkyl or alkylene of C8-C30 as a branched or straight carbon chain, or C12-C30 aralkyl, and may be optionally substituted with 0-100 alkylene oxide groups such as ethylene oxide, propylene oxide groups, oligameric lactic and/or glycolic acid or a combination thereof; and
   M is H, an alkali metal, an alkaline earth metal, or an ammonium group.
4. The fine fiber of item 3, wherein the ammonium group is a protonated tertiary or quaternary amine.
5. The fine fiber item 3 wherein the ammonium group is a protonated triethanolamine or tetramethylammonium.
6. The fine fiber of item 3, wherein M is an alkali metal or alkaline earth metal.
7. The fine fiber of item 3, wherein M is selected from the group consisting of calcium, sodium, potassium, or magnesium.
8. The fine fiber of item 1, wherein the viscosity modifier is selected from the group consisting of stearoyl lactylates and stearates.
9. The fine fiber of any of the preceding items, wherein the viscosity modifier is present in an amount less than about 10 percent by weight based on the total weight of the fiber.
10. The fine fiber of any of the preceding items, further comprising a thermoplastic polymer distinct from the thermoplastic aliphatic polyester.
11. The fine fiber of any one of the previous items, wherein the viscosity modifier is present in an amount less than 2% by weight.
12. The fine fiber of any of the preceding itmes, wherein the viscosity modifier comprises less than 5% water.
13. The fine fiber of any of the preceding items, further comprising a surfactant distinct from the viscosity modifier.
14. The fine fiber of any of the preceding items, wherein the aliphatic polyester is semicrystalline.
15. The fine fiber of any of the preceding items, further comprising an antimicrobial component.
16. The fine fiber of any of the preceding items, wherein the composition is biocompatible.
17. The fine fiber of any of the preceding items, wherein the composition is melt processable.
18. An article comprising the composition of any of the preceding items, said article being selected from molded polymeric articles, polymeric sheet, polymeric fibers, woven webs, nonwoven webs, porous membranes, polymeric foams, thermal or adhesive laminates, and combinations thereof.
19. The article of item 18, wherein the nonwoven is selected from the group consisting of a spunbond web, a blown microfiber web, or a hydroentangled web.
20. A surgical drape comprising the fine fibers of any of items 1 to 17.
21. A surgical gown comprising the fine fibers of any of items 1 to 17.
22. A wound contact material comprising the fine fibers of any of items 1 to 17.
23. A personal hygiene article comprising the fine fibers of any of items 1 to 17.
24. A sterilization wrap comprising the fine fibers of any of the preceding items 1 to 17.
25. A method of making fine fibers, comprising
   providing an aliphatic thermoplastic polyester;
   providing a viscosity modifier selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof; and
   mixing the aliphatic plyester and the viscosity modifier; and
   forming fibers from the mixture.
26. The method of item 25 wherein the fibers are formed using a melt-blowing, spun-bonding, or melt-spinning process.
27. The method of item 25, wherein the fibers form of a nonwoven web.
28. The method of item 25, wherein the aliphatic thermoplastic polyester and the viscosity modifier are mixed prior to the fiber forming process.
29. The method of any of the preceding items, further comprising the step of extruding the aliphatic polyester blended with the viscosity modifier.
30. The method of any one of the preceding items, wherein the mixing of the aliphatic polyester and the viscosity modifier comprises extruding the aliphatic polyester and the viscosity modifier.
31. The method of any of the preceding items, further comprising post healing the web.

### EXAMPLES

The polymer resin used in the examples is 6251D PLA available as pellets from Natureworks, LLC, Minnetonka, Minnesota. Natureworks reports 6215D PLA to have a relative viscosity of 2.50 and a d-isomer content of 1.4%. Using GPC, the molecular weight of the resin was found to be 94,700 daltons for Mw, and 42,800 daltons for Mn. Calcium Stearoyl Lactylate (CSL) is available commercially as Pationic CSL from RITA Corp. (Crystal Lake, Illinois) as a cream colored powder.

### EXAMPLES 1-2

CSL was added to the system in the concentrations shown in Table 1 by dry blending the CSL powder with warm PLA pellets from the polymer dryer. The resin was predried by heating to 71 C overnight. The CSL melted on contact with the warm PLA pellets and was blended by hand to form slightly sticky pellets that were then fed to the extruder.

For examples 1-2 and the control, die temperature was held at 225°C and all other process conditions were held constant. The pump exit pressure measured the entire pressure drop of the polymer stream through the die and the necktube.

Example 2 with 2.0% CSL produced a small amount of polymer particles along with the fibers. This phenomenon is referred to as "sand", and is a common flaw in BMF processing.

It was found that adding the CSL to neat PLA resin before or during extrusion greatly reduced the pressure drop across the die as shown in Table 1. It was also noted that the fiber diameter decreased significantly as well. After aging the squares were measured and the average linear shrinkage is also reported in Table 1.

**Table 1:**

| Example | Material | Pump Exit Pressure (psi) | Eff. Fiber Diameter (microns) | 80°C Shrinkage (linear %) |
|---|---|---|---|---|
| Control | Neat 6251D PLA | 671 | 19.6 | 14.25 |
| 1 | 1.0% CSL in 6251D | 372 | 11.4 | 24.70 |
| 2 | 2.0% CSL in 6251D | 262 | 9.7 | 10.08 |

The finer diameter webs are noticeably softer and more conformable compared to the control sample. The shrinkage of the webs that included CSL was substantial.

### EXAMPLES 3-5

CSL was pre-blended at high concentration prior to fiber formation. This high concentration mixture is commonly called a masterbatch. The masterbatch is typically dry blended with neat polymer pellets when feeding to the fiber extruder. The extrusion process then provides additional mixing.

A masterbatch of 10% CSL in 6251D PLA was prepared on a twin screw extruder, cooled as strands in a water bath, then pelletized using a dry pelletizer. The solid pellets were dried in an 80°C oven overnight to remove any trace water from the water bath.

Melt-blown fibers were extruded using the same equipment as Example 1. Again extrusion temperature was held at 225°C. Four CSL samples were produced with final concentrations of CSL and the results shown in Table 2.

**Table 2:**

| Example | Material | Pump Exit Pressure (psi) | Eff. Fiber Diameter (microns) | 80°C Shrinkage (linear %) |
|---|---|---|---|---|
| Control | Neat 6251D PLA | 431 | 16.8 | 3.16 |
| 3 | 0.5% CSL in 6251D | 142 | 11.7 | 13.91 |
| 4 | 0.75% CSL in 6251D | 122 | 11.1 | 8.50 |
| 5 | 1.0% CSL in 6251D | 62 | 8.8 | 17.50 |

The 0.75% and 1.0% samples exhibited some sand in the finished webs.

The pump exit back pressure dropped precipitously with minor additions of calcium stearoyl lactylate (Pationic CSL), as shown in Fig.1. A regression analysis of the data shows that the data fits the following second order polynomial Melt viscosity = 351 (Pationic concentration)² -706 (Pationic concentration) + 428 where r² = 0.985. Where Pationic concentration is in weight % and melt viscosity is expressed as the pump exit pressure in PSI (1bs/in²).

The polynomial shows that the viscosity modifier dramatically effects melt viscosity.

### EXAMPLES 6-11

Other fatty salts have shown to be effective in reducing fiber diameter along with CSL. For these experiments, various powdered salts in the concentrations shown in Table 3 were dry blended with neat PLA pellets prior to extrusion. The additives tested included:
Sodium Stearoyl Lactylate (SSL) (PATIONIC SSL, from RITA Corp.) -as an off-white powder
Calcium Stearate (Ca-S) (Aldrich, St. Louis, MO)
Sodium Behenoyl Lactylate (SBL) (PATIONIC SBL, from RITA Corp.) as an off-white colored powder
Examples 6-11 were run on slightly larger equipment such that pressure measurements were not directly comparable between the two pieces of equipment. The operating temperature was held constant at 210°C. The results of the experiment are shown in Table3. During the extrusion run the pump exit pressure sensor failed, and no reading was taken for the control sample.

All three of these salts (SSL, SBL, Ca-S) produced webs that contained larger amounts of sand than CSL, which gave the webs a very rough feel. However despite the sand, both additives substantially reduced the fiber diameter of the melt-blown webs.

**Table 3**

| Example | Material | Pump Exit Pressure (psi) | Eff. Fiber Diameter (microns) | 80°C Shrinkage (linear %) |
|---|---|---|---|---|
| Control | Neat 6251D PLA | Failed sensor | 25.8 | 6.0 |
| 6 | 1% SSL in 6251D | 744 | 16.7 | 17.75 |
| 7 | 1.5% SSL in 6251D | 968 | 15.5 | 9.75 |
| 8 | 2% SSL in 6251D | 425 | 12.7 | 29.0 |
| 9 | 2% SBL in 6251D | 69 | 5.5 | 19.25 |
| 10 | 1% Ca-S in 6251D | 83 | 10.0 | 10.25 |
| 11 | 2% Ca-S in 6251D | 44 | 8.0 | 23.08 |

While certain representative embodiments and details have been discussed above for purposes of illustrating the invention, various modifications may be made in this invention without departing from its true scope, which is indicated by the following claims.

## Claims

1. A single or multi-layer aqueous repellent article formed at least in part of a web of fine fibers, wherein the fine fibers comprise:
one or more thermoplastic aliphatic polyesters; and
a viscosity modifier selected from the group consisting of alkyl carboxylates, alkenyl carboxylates, aralkyl carboxylates, alkylethoxylated carboxylates, aralkylethoxylated carboxylates, alkyl lactylates, alkenyl lactylates, and mixtures thereof; and
wherein the fine fibers have an average fiber diameter of less than 20 microns; and
wherein a repellent additive is incorporated into the melt as the web is made, a repellent additive is coated onto the fibers prior to web formation, or a repellent additive is coated onto the formed or semi-formed web.

2. The article of claim 1, wherein the repellent additive is incorporated into the melt as the web is made.

3. The article of claim 1, wherein the repellent additive is coated onto the fibers prior to web formation.

4. The article of claim 1, wherein the repellent additive is coated onto the formed or semi-formed web.

5. The article of claim 4, wherein the repellent additive is selected from paraffin waxes, fatty acids, bee's wax, silicones, fluorochemicals and combinations thereof.

6. The article of claim 1, wherein the repellent additive is a fluorochemical compound containing one or more fluoroaliphatic radicals, wherein the fluorochemical compound is selected from fluorochemical urethanes, ureas, esters, amines and salts thereof, amides, acids and salts thereof, carbodiimides, guanidines, allophanates, biurets, and compounds containing two or more of these groups, as well as blends of these compounds.

7. The article of claim 6, wherein the repellent additive is a fluorochemical polymer containing one or more fluoroaliphatic radicals, wherein the fluorochemical polymer is selected from copolymers of fluorochemical acrylate and/or methacrylate monomers with co-polymerizable monomers.

8. The article of claim 1, wherein the fine fibers have an average fiber diameter of less than 5 microns.

9. The article of any of the preceding claims, wherein the aliphatic polyester is semicrystalline.

10. The article of claim 9, wherein the one or more thermoplastic aliphatic polyesters comprise a poly(lactic acid) that is at least 85% of one isomer.

11. The article of claim 1, wherein the viscosity modifier has the following structure:
(R-CO₂⁻)ₙMⁿ⁺
wherein R is an alkyl or alkylene of C8-C30 as a branched or straight carbon chain, or C12-C30 aralkyl, and may be optionally substituted with 0-100 alkylene oxide groups, oligomeric lactic and/or glycolic acid or a combination thereof;
M is H, an alkali metal, an alkaline earth metal, or an amine group; and
n is the valency of M.

12. The article of claim 1, wherein the viscosity modifier is selected from the group consisting of stearoyl lactylates and stearates.

13. The article of any of the preceding claims, wherein the fine fibers further comprise a thermoplastic polymer distinct from the thermoplastic aliphatic polyester.

14. The article of any one of the previous claims, wherein the viscosity modifier is present in an amount less than 2% by weight based on the combined weight of the aliphatic polyester and viscosity modifier.

15. The article of any of the preceding claims, wherein the fine fibers further comprise an antimicrobial component.

16. The article of any of the preceding claims, wherein the fine fiber composition is biocompatible.
